Europäisches Patentamt

European Patent Office

Office européen des brevets

(11) Publication number: **0 476 992 B1**

## EUROPEAN PATENT SPECIFICATION

(45) Date of publication of patent specification: **19.07.95** (51) Int. Cl.⁶: **A61F 13/62**

(21) Application number: **91308511.4**

(22) Date of filing: **18.09.91**

(54) **Mechanical fastener and diaper construction.**

(30) Priority: **21.09.90 US 586574**

(43) Date of publication of application:
**25.03.92 Bulletin 92/13**

(45) Publication of the grant of the patent:
**19.07.95 Bulletin 95/29**

(84) Designated Contracting States:
**DE ES FR GB IT**

(56) References cited:
**EP-A- 0 336 639**
**EP-A- 0 393 953**
**FR-A- 2 389 348**
**US-A- 4 699 622**

(73) Proprietor: **MINNESOTA MINING AND MANU-
FACTURING COMPANY
3M Center,
P.O. Box 33427
St. Paul,
Minnesota 55133-3427 (US)**

(72) Inventor: **Melbye, William L., c/o Minnesota
Mining and
Manufact. Co.,
2501 Hudson Road,
P.O. Box 33427
St. Paul,
Minnesota 55133-3427 (US)**

(74) Representative: **Baillie, Iain Cameron et al
c/o Ladas & Parry
Altheimer Eck 2
D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

Technical Field

This invention relates to a high shear strength mechanical fastener for use in preventing shifting of foraminous substrates. Particularly, the invention relates to a mechanical fastener for use in garments such as disposable diapers to prevent slippage of the garment adjacent a conventional closure.

Background Art

Garments and disposable garments, such as diapers and related articles, are well known. For example, a conventional type of diaper system used by both infants and incontinent adults is comprised of an absorbent core encased between a liquid permeable "top sheet" (the user contacting face) and a liquid-impermeable "back sheet" (the outer shell portion), which generally rectangular shaped composite encircles the wearer's waist in association with closure systems. These closure systems are most frequently located so that they join front and rear ends or panels of the diaper. These closure systems are most commonly located at either side of the wearer to join opposing corners of the diaper. In order to improve the fit of these diapers and prevent leakage, the side edge portions of the diapers are frequently elasticized which, when the diaper is joined by the side closure systems, provide elasticized leg openings which grip the wearer's thighs.

Conventional diaper closure systems are discussed in U.S. Patent No. 4,699,622 (Toussant et al.). Toussant et al. was concerned with the problem of diapers shifting on the wearer when used. More specifically, Toussant et al. stated this problem as where

"overlapping front and back waist portions were subjected to forces which tend to cause the front and back waist portion to assume a position relative to each other which is different from the position they assume when a diaper is initially fitted to the wearer."

Toussant et al.'s proposed solution to this problem is a two point closure system comprised of a conventional type "outer fastening means", which fixes overlapping corners of the front and rear diaper panels to each other. This outer fastening is preferably done with an adhesive fastening tab that will releasably attach to the diaper "backsheet", preferably on a front panel located at the waist engaging portion or area of the diaper. In addition to this outer fastening means, Toussant et al. proposes the use of an "inner fastening means" to prevent shifting of the overlapping corners of the diaper, each with respect to the other, from wearer movement forces and forces from the elasticized portions of the diaper. Preferably, the inner fastening means is disclosed as a mechanical type engaging material which is fixed to backsheet corners, at the front panel portion of the diaper, and which is capable of entangling with, e.g., fibrous material typically used as the porous topsheet. The preferred material disclosed is that marketed by 3M Company, St. Paul, Minnesota under the trade name SJ-3492.

Conventional mechanical fasteners are quite costly and are designed to engage loose weave loops or the like to provide significant peel force resistance. A diaper inner liner is conventionally formed of a non-woven material. Although some non-woven materials can be engaged by most conventional mechanical fasteners, the fibers must have a fairly open structure to do so efficiently. However, such a loose or open non-woven is not necessarily as effective in preventing contact of the diaper wearer with the absorbent core material.

As described in Toussant et al., a preferred non-woven is carded, then thermally bonded. This process can make the non-woven difficult to penetrate with conventional mechanical fasteners, which have a relatively large cross sectional profile at their tips. If penetration is possible, often higher application pressures are required to penetrate closely associated non-woven fibers with a conventional mechanical fastener. Using high application pressures is obviously difficult with an infant diaper and as such conventional mechanical fasteners are not likely to be effectively applied by the person fitting the diaper with preferred diaper topsheets.

Summary of the Invention

In accordance with the invention, there is provided a diaper as claimed in claim 1 herein.

The diaper fastener is formed of a backing having an array of upstanding stemlike projections distributed across at least one face. The stemlike projection tips are substantially pointed to allow for easy penetration into a foraminous substrate, such as is used as a diaper topsheet.

This high shear strength fastener finds particular use in a disposable diaper, as is disclosed in Toussant et al., as an inner fastener means. An inner fastener means formed from the high shear strength mechanical fastener is adapted to readily engage at least the top sheet of the diaper, which is a non-woven material. Coupled with a conventional outer fastening means, this inner fastener will provide a secure two-point closure less likely to shift or twist on the wearer.

Brief Description of the Drawings

Fig. 1 is a schematic view of a disposable diaper from the top sheet side.
Fig. 2 is a schematic view of a disposable diaper similar to that of Fig. 1 as it would appear while being worn.
Fig. 3 is a schematic isometric view of a fastener in a partial cut away vertical cross section.
Fig. 4 is a schematic isometric view of a fastener in a partial cut away vertical cross section.
Fig. 5 is a plan view of a fastener of the present invention.

Detailed Description of the Preferred Embodiments

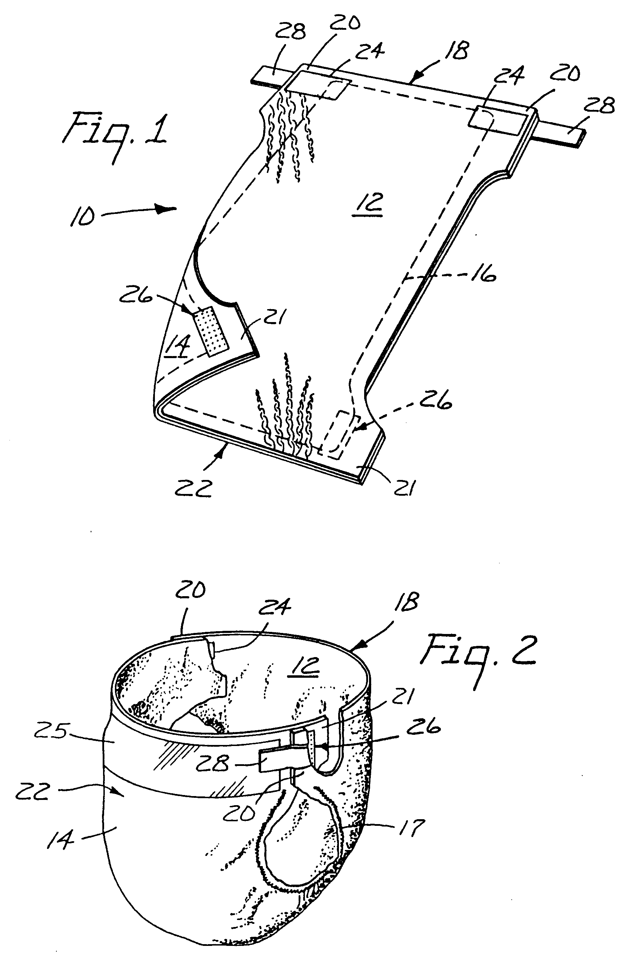

Referring to the drawings, there is shown a preferred embodiment of the present invention used in a disposable diaper, such as would be worn by an infant or an incontinent adult. The disposable diaper 10 shown in Fig. 1 would conventionally be at least a three-layer composite including a liquid permeable, user contacting top sheet 12, a liquid-impervious outer shell or back sheet 14 and an absorbent layer 16. At the back panel 18 of the diaper are corners 20 that overlap with corresponding corners 21 at the front panel 22 of the diaper when the diaper is worn. On the top sheet 12 side of the diaper at each of the corners 20 is located a release treated, non-woven release tab 24 and on the outer shell or back sheet 14 at the front corners 21 of the diaper 10 are mechanical type fasteners 26 of the invention.

For most conventional designs, the fastening tabs 28 are located at the back panel 18 attached at least to the back sheet 14 of the diaper 10. During non-use, the tabs 28 would be located on the non-woven release treated tabs 24. When in use, the fastening tabs 28 would be removed from the release treated non-woven tabs 24 and attached to the front panel 22 of the diaper back sheet 14. Generally, the diaper front panel 22 is provided with a landing or frontal strip 25, as shown in Fig. 2, which reinforces the diaper at the waist portion of the diaper front panel 22, allowing removal and replacement of the fastening tab as necessary. The side edge portions of the diaper are also provided with elasticizing elements 17, also shown in Fig. 2, which provide for engaging the leg or thigh of the wearer when the diaper is in use. The diaper can be constructed by any conventional means, as is disclosed in Toussant et al.

Conventionally, the top sheet 12 of the diaper is a soft, non-irritating fabric to prevent irritation of the wearer's skin. Top sheet fabric is generally fibrous woven or non-woven sheets formed of, e.g., natural fibers, such as cellulose, or synthetic fibers of polyester, polyethylene, or polypropylene or a combination thereof. Further, the fibers may or may not be treated or contain additives depending upon the surface characteristics of the fiber and the desired properties of the top sheet. Other materials capable of passing moisture to the absorbent inner layer 16 are also possible, such as a formed film or foam, e.g., a reticulated foam, as is known in the art.

The top sheet 12 can be formed by any suitable method including woven manufacturing such as weaving, and non-woven manufacturing such as spinbonding, carding, etc.

Fastening tab 28 will generally be permanently fixed to back sheet 14 at corners 20, although other placements are possible, by any suitable method. Pressure-sensitive adhesives are preferred to fix the tab 28 to the back sheet 14. However, in addition to using pressure-sensitive adhesives, the fastening tabs 28 can be directly heat sealed to the back sheet 14 or adhered using a heat or solvent activated adhesive, depending upon the nature of the back sheet. The mechanical fastener 26 and release tabs 24 (if separately attached) can similarly be attached by any conventional method as described above with respect to the fastening tabs. The opposite end of the fastening tab 28 will be releasably attached to the release tabs 24 when not in use and removed by the user for placement on, e.g., the frontal strip 25 on the back sheet 14 front panel 22 portion. This will cause overlap of corners 20 and 21. Generally, the user or free end of the fastening tab 28 is provided with a pressure-sensitive adhesive which will releasably adhere to the front panel portion.

In accordance with the above described preferred embodiment, the inner fastening means is provided on the back sheet 14 of the diaper at corners 21, which inner fastening means comprises the mechanical fastening means 26. This mechanical fastening means 26 is provided to primarily resist sheer forces which may cause the overlapping corners 20 and 21 to shift relative to each other when the diaper or incontinence article is worn.

The release tab 24 shown is located on the top sheet side of corner 20. The top sheet 12 face of corner 20 will overlap with the back sheet face of corner 21 when the diaper is assembled as shown in Fig. 2.

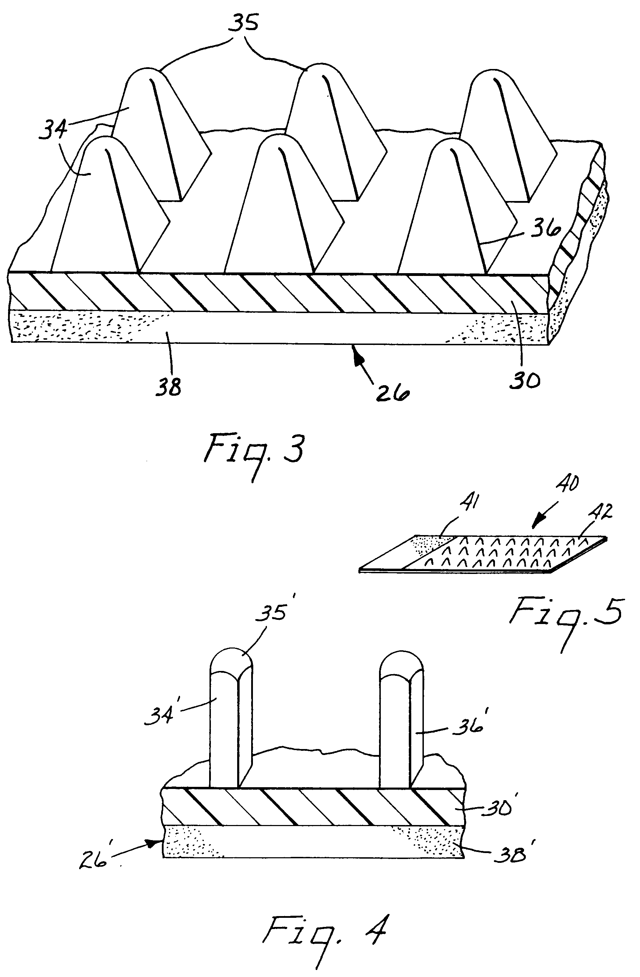

The mechanical fastening tab of the invention is designed to permit ready penetration into non-woven and other foraminous substrates, as would likely be used as a diaper topsheet, or in like articles or garments. The mechanical fastener depicted in Fig. 3 comprises a backing 30, which is preferably flexible. From at least one face of the backing projects an array of upstanding stems or projections 34 having, generally, blunt or pointed tips 35. The projections as shown are in rows, however, any suitable arrangement can be employed which will permit ready penetration into the foraminous substrate being used.

The stems 34 and the backing 30 are preferably flexible, with the stems somewhat resistant to compression or bending when in use. Materials suitable for forming this integral structure include substantially any thermoplastic material useful in the production of films. Preferred of such thermoplastic materials are tough thermoplastic resins of polyethylene, polypropylene, polyesters, polyamides (e.g., nylon), and copolymers thereof. These materials can be used to form the mechanical fastener by any suitable method including cast or extrusion molding.

The backing can be quite thin depending on the application. Generally, a thickness of about $25\mu m$ is needed to prevent breakage during use. Thicker backings can be used depending on the particular end use and the strength required therefore. Generally, thinner backings are preferred for disposable diapers and the like in terms of material flexibility, conformability and cost.

The stems can be of any shape which permits ready penetration into a foraminous substrate. This shape is preferably one which has an inward taper, see Fig. 4 where like numerals (26', 30', 34', 35', 36' and 38') indicate like elements discussed with respect to Fig. 3, away from the backing face, such as a pyramidal or conical shape. However, a shape with a slight inward or outward taper is contemplated if the shape does not significantly interfere with substrate penetration by the stem tip 35. Significant outward tapers or traditional mechanical fastener structures at the top of the stems are not desired as they complicate fabrication and are not required for the invention high shear strength fastener. The tip 35 mean diameter is generally from 0.5 to 15 mils (12.7 to 381 $\mu m$) for use on a standard diaper non-woven material, with 2 to 8 mils (51 to 203 $\mu m$) being preferred. The mean diameter of the stems at their base 36 is generally 1.5 to 20 mils (38 to 508 $\mu m$), with 4.5 to 12 mils (114 to 305 $\mu m$) being preferred. With preferred mean stem diameters, a minimum of approximately 25 stems per square inch (3.9 stems/cm$^2$) is preferred, with up to 2,500 stems per square inch (388 stems/cm$^2$) having been demonstrated as feasible, with a minimum of approximately 50 stems per square inch (7.75 stems/cm$^2$) being most preferred. However, use of over at least 1,000 stems/in$^2$ (155 stems/cm$^2$) is generally not preferred as there is no significant increase, and generally a slight decrease, in performance over this stem density. Further, theoretically, performance will decrease significantly at extremely high stem densities due to adjacent stem interference with fiber penetration. The lower stem densities, although functional, are not generally as desirable due to their coarse feel. The higher the stem density, the less noticeable is the fastener when in contact with the human body. An overall preferred range of stem densities would be from 75 to 1,000 stems/in$^2$ (10.65 to 155 stems/cm$^2$).

Stem height is also important, with a height of 5 to 20 mils (130 to 510 $\mu m$) being preferred, and with 10 to 15 mils (250 to 380 $\mu m$) being more preferred. Stem heights lower than the preferred minimum do not easily penetrate foraminous substrates, specifically non-woven materials. A stem height above the preferred maximum will have a tendency to bend when subjected to shear forces, particularly when the stems do not fully penetrate the foraminous substrate. Further, higher stems do not offer any significant performance increase for the added costs associated with their manufacture. A fastener formed of the stem-faced material preferably will be of an overall size such that it will provide a shear force resistance of at least 500 grams, and preferably at least about 750 grams, when placed against a foraminous substrate at a pressure of approximately 17 grams/cm$^2$. The fastener size is limited only by the foraminous substrate available for attachment. The fastener, however, is preferably small in terms of cost, conformability to the wearer, wearer comfort and ease of use.

The back face of the mechanical fastener is preferably substantially flat to permit application of an adhesive layer 38 for attachment purposes. An advantage with the invention fastener material when using an adhesive layer 38 is that the stems permit the adhesive backed fastening material to be formed into a roll prior to fabrication. The stems present a relatively low surface area available for adhesive contact such that the material can be wound as a roll then unwound without the necessity of treating the stem face with a low adhesion backsize or release coating. This allows for convenient manufacture, storage and shipment of the bulk fastening material prior to formation and assembly of the mechanical fastener on the garment (e.g., such as a diaper as described above).

The mechanical fastener can be used in other garments for purposes similar to that in the diaper configuration described above. Generally, where a garment or the like requires a fastener with high shear resistance and little or no peel resistance, this mechanical fastener could find use. Further, the fastener (42) could be used in close conjunction with a conventional adhesive or mechanical fastener to provide shear enhancement. The mechanical fastener 40 (Fig. 5), in this case, could be integral with or closely adjacent the conventional fastener 41 such as on a separate area of the same backing.

The following non-limiting examples serve to illustrate the invention, however, are not intended to be limiting thereof.

Example 1

One piece (2 in. by 3 in. (5.1 x 7.6 cm)) of film, 4.5 mil (114 $\mu$m) thick, prepared by cast extrusion of a polypropylene homopolymer resin (Fina Dypro™ 8771, 9 melt flow index) was placed on a metal plate which had the negative impression to produce a stem geometry and pattern as illustrated in Fig. 3 of the drawings. A flat metal plate was placed on top of the film. This was pressed in a platen press at 2500 psi (176 kg/cm) and 330°F (151°C) for 5 seconds. The press was then opened for 5 seconds, then closed again for 15 seconds. Then, after air cooling for 20-30 minutes, the embossed film was removed. Physical dimensions of the stems are reported in Table I.

Table 1

| Spacing Sample | Stem Height ($\mu$m) | Stem Width at Base ($\mu$m) | Stem Density (per/cm$^2$) | Center-to-Center Stem ($\mu$m) [2] |
|---|---|---|---|---|
| 1. | 152 | 189 | 25.1 | 2,145 |
| 2. | 203 | 202 | 12.9 | 3,002 |
| 3. | 203 | 202 | 37.0 | 1,766 |
| 4. [1] | 203 | 150 | 387.5 | 500 |
| 5. | 254 | 214 | 62.0 | 1,250 |
| 6. | 254 | 214 | 172.2 | 750 |
| 7. [3] | 330 | 233 | 4.1 | [4] |
| 8. | 330 | 233 | 8.2 | 3,753 |
| 9. | 330 | 233 | 25.1 | 2,145 |
| 10. | 330 | 233 | 41.5 | 1,668 |
| 11. | 457 | 264 | 12.9 | 3,002 |
| 12. | 457 | 264 | 37.0 | 1,766 |
| 13. | 508 | 276 | 25.1 | 2,145 |

(1) Sample 4 was drilled with a 4 mil (101 $\mu$m) stem tip while all others incorporated a 6 mil (152 $\mu$m) tip.
(2) Samples 1-3 and 8-13 were drilled with staggered holes with uniform spacing between holes. Samples 4-6 were drilled in perpendicular rows with uniform spacing between rows.
(3) Sample 7 was created by removing every second stem on sample 8.
(4) This sample did not have uniform stem spacing due to the fact that it was produced by removing every second stem from a staggered pattern.

Example 2

Molten resin (Dypro™ 9618, an ethylene propylene copolymer from Fina Oil and Chemical Co., Dallas, Texas), was continuously cast onto a rotating steel forming roll using a standard single screw extruder. The forming roll had an array of holes drilled in it representing the negative of a desired projection geometry and spacing. The holes were formed in the forming roll with Minitool microdrilling heads, available from Minitool, Inc., Campbell, CA. To facilitate flow of the molten polymer into the holes, the surface of the forming roll was exposed to a vacuum 636 N/m$^2$ (46.8 mm of mercury) during the casting process using a vacuum chamber preceding and attached to the extrusion die and seated directly on the forming roll. The temperature of the forming roll was maintained at about 35°C by standard means of internal roll cooling with circulating water.

A gap was provided between the extrusion die and the forming roll to allow sufficient molten resin to be applied to the forming roll to fill the holes and provide a backing integral with the resulting projections when the quenched resin was stripped off of the forming roll to yield the backing of Example 2. The stems were pyramidal with approximately 26 stems/cm$^2$, a stem height of 760 microns, a stem width at the base of 410 microns and a stem spacing center-to-center of 1,270 microns.

Example 3

Various of the materials were tested for shear resistance in accordance with the method outlined in U.S. Patent No. 4,699,622. The samples, 2.5 in. x 2.5 in. (6.35 cm x 6.35 cm, 40 cm$^2$), were placed on a friction sled. A piece of foam and the non-woven, with the foam underneath, were taped to the friction platform of the peel tester (Instrumentors, Inc. Model 3M-90). The non-woven was a spunbond polypropylene with a basis weight of 60 gm/m$^2$ purchased from James River Corporation. The sled was then run over the platform and a shear value was obtained. A baseline value was obtained by placing standard diaper polyethylene on the sled. The wrapped sled was used alone, which had a weight of approximately 200 gm (5 gm/cm$^2$), and with additional weights, for a total of approximately 700 gm (17 gm/cm$^2$). For the 700 gram weighted sled, this baseline shear value was 3.9 N/40 cm$^2$ (400 grams/40 cm$^2$), and for the 200 gram sled, the baseline shear was 1.25 N/40 cm$^2$ (128 grams/40 cm$^2$). The measured results were translated to what would be obtained with a 1 in. x 2.5 in. (2.54 cm x 6.35 cm) sample by the following equation:

$$\text{base line value} \times \frac{(40.3\ cm^2 - \text{sample size})}{40.3\ cm^2}$$

$$\underbrace{\text{test result}}_{\uparrow}$$

$$\frac{16.13\ cm^2}{\text{sample size}}$$

The shear resistance obtained for the tests run with the sled and the weighted sled are set forth in Table 2.

EP 0 476 992 B1

Table 2

| Sample | Sled Shear (gm) | Weighted Sled Shear (gm) |
|--------|-----------------|--------------------------|
| 1. | 389 | 691 |
| 2. | 471 | 828 |
| 3. | 461 | 856 |
| 4. | 698 | 1,133 |
| 5. | 578 | 1,011 |
| 6. | 701 | 1,280 |
| 7. | 263 | 683 |
| 8. | 463 | 868 |
| 9. | 514 | 888 |
| 10. | 463 | 1,093 |
| 11. | 419 | 695 |
| 12. | 556 | 745 |
| 13. | 449 | 896 |

Other embodiments of the invention will be apparent to those skilled in the art from consideration of the specification or practice of the invention disclosed herein. It is intended that the specifications and examples be considered as exemplary, with the true scope of the invention being defined by the following claims.

**Claims**

1. A diaper (10) comprising a liquid-permeable nonwoven topsheet (12) and a liquid-impermeable backsheet (14) attached to said topsheet (12) with an absorbent batt (16) between said topsheet (12) and said backsheet (14), said diaper (10) having longitudinal side edges, at least two opposed corner portions (20 and 21) along each side edge, which at least two opposing corner portions (20 and 21) overlap when the diaper is worn, at least one outer fastening means (28) permanently attached closely adjacent one side edge of said diaper at a one corner portion (20) of said at least two opposing corner portions (20 and 21) for securing said one of said at least two corner portions (20 and 21) in overlapping arrangement with the other corner portion (21) of said at least two corner portions (20 and 21) by removable attachment of said at least one outer fastening means (28) on said one corner portion (20) of said at least two corner portions (20 and 21) to a front panel portion (22) of said backsheet adjacent the said other corner portions (21), and at least one inner fastening means (26) permanently attached to said backsheet (14) adjacent said one side edge of said diaper (10) within only said other corner portion (21) for securing the nonwoven topsheet (12) of said one corner portion (20) to said backsheet (14) of said other corner portion (21), said at least one inner fastening means (26) comprising a backing (30, 30') having two faces, one face attached to said backsheet (14) and a second face characterized in that the second face has a stem(36, 36')-containing region comprising an array of upstanding thermoplastic stems (36, 36') distributed across at least said second face, said stems terminating in a tip means (35, 35') to penetrate said nonwoven topsheet (12), said stems (36, 36'), including said tip means (35, 35'), having no projections thereon for gripping fibers, said at least one inner fastening means located on the backsheet such as to allow penetration of said nonwoven topsheet (12) or a foraminous (24) substrate on said nonwoven topsheet at said one corner portion (20) by said stems (36) of said stem-containing region when said at least one outer fastening means (28) is attached to said front panel portion (22).

2. The diaper of claim 1 characterized in that said stems (36, 36') have an inward taper away from said second backing (30, 30') face.

3. The diaper of claim 2 characterized in that said stems (36) are substantially conical or pyramidal.

4. The diaper of claim 1 characterized in that said stems (36) have substantially no taper (34') away from said second backing face.

5. The diaper of claim 1 characterized in that said stems (36, 36') are 130 to 510 $\mu$m from said tip means (35, 35') to the plane of the said second backing (30, 30') face.

8

EP 0 476 992 B1

**6.** The diaper of claim 1 wherein said inner fastening means (26) comprises a mechanical and adhesive fastener (40) said second face comprises an adhesive-containing region (41) and the stem-containing region (42) is adjacent to said adhesive-containing region (41).

**Patentansprüche**

**1.** Windel (10) mit einem flüssigkeitsdurchlässigen Innenblatt (12) aus Vliesstoff und einem flüssigkeitsundurchlässigen Außenblatt (14), das an dem Innenblatt (12) angebracht ist, wobei zwischen dem Innenblatt (12) und dem Außenblatt (14) saugfähige Watte (16) vorgesehen ist, die Windel (10) Längsseitenränder und längs jedes Seitenrandes mindestens zwei einander entgegengesetzte Eckteile (20 und 21) besitzt, die beim Tragen der Windel einander überlappen, ferner mindestens ein äußeres Schließmittel (28), das in der Nähe eines Seitenrandes der Windel an einem (20) der mindestens zwei einander entgegengesetzten Eckteile (20 und 21) dauerhaft angebracht ist und dazu dient, den mindestens einen (20) der mindestens zwei Eckteile (20 und 21) an dem anderen (21) der mindestens zwei Eckteile (20 und 21) überlappend zu befestigen, indem das mindestens eine an dem einen (20) der mindestens zwei Eckteile (20 und 21) vorgesehenen äußeren Schließmittel (28) an dem Vorderteil (22) des Außenblattes im Bereich des genannten anderen Eckteils (21) lösbar angebracht wird, sowie mindestens ein Inneres Schließmittel (26) das an dem Außenblatt (14) im Bereich des genannten einen Seitenrandes der Windel (10) nur innerhalb des genannten anderen Eckteils (21) dauerhaft angebracht ist und dazu dient, das aus Vliesstoff bestehende Innenblatt (12) des genannten einen Eckteils (20) an dem Außenblatt (14) des genannten anderen Eckteils (21) zu befestigen, wobei das mindestens eine innere Schließmittel (26) einen Rücken (30, 30') mit zwei Oberflächen aufweist, und zwar mit einer an dem Aussenblatt (14) angebrachten Oberfläche und einer zweiten Oberfläche, dadurch gekennzeichnet, daß die zweite Oberfläche einen Zapfen (36, 36') enthaltenden Bereich mit einer Anordnung von aufwärtsgerichteten thermoplastischen Zapfen (36, 36') besitzt, die mindestens über die zweite Oberfläche verteilt sind und in je einem Scheitel (35, 35') enden, der das aus Vliesstoff bestehende Innenblatt (12) durchdringen kann, wobei die Zapfen (36, 36') einschließlich der Scheitel (35, 35') keine Vorsprünge zum Erfassen von Fasern besitzen und das mindestens eine innere Schließmittel auf dem Außenblatt derart angeordnet ist, daß die Zapfen (36) des die Zapfen enthaltenden Bereichs durch das aus Vliesstoff bestehende Innenblatt (12) oder auf dem aus Vliesstoff bestehenden Innenblatt an dem genannten einen Eckteil (20) hindurchtreten können, wenn das mindestens eine äußere Schließmittel (28) an dem genannten Vorderteil (22) angebracht ist.

**2.** Windel nach Anspruch 1, dadurch gekennzeichnet, daß die Zapfen (36, 36') von der genannten zweiten Oberfläche des Rückens (30, 30') weg einwärts verjüngt sind.

**3.** Windel nach Anspruch 2, dadurch gekennzeichnet, daß die Zapfen (36) im wesentlichen kegel- oder pyramidenförmig sind.

**4.** Windel nach Anspruch 1, dadurch gekennzeichnet, daß die Zapfen (36) von der zweiten Oberfläche des Rückens weg im wesentlichen keine Verjüngung (34') besitzen.

**5.** Windel nach Anspruch 1, dadurch gekennzeichnet, daß die Zapfen (36, 36') von dem Scheitel (35, 35') zu der Ebene der zweiten Oberfläche des Rückens (30, 30') 130 bis 510 Mikrometer messen.

**6.** Windel nach Anspruch 1, in der das innere Schließmittel (26) einen mechanischen Klebeverschluß (40) aufweist, die zweite Oberfläche einen klebstoffhaltigen Bereich (41) besitzt und der Zapfen enthaltende Bereich (42) dem klebstoffhaltigen Bereich (41) benachbart ist.

**Revendications**

**1.** Couche culotte (10) comprenant un voile supérieur non tissé (12) perméable au liquide et un voile arrière (14) imperméable au liquide associé au voile supérieur (12), un matelas absorbant (16) étant prévu entre ledit voile supérieur (12) et ledit voile inférieur (14), ladite couche culotte (10) ayant des bords latéraux longitudinaux, au moins deux parties de coin opposés (20 et 21) le long de chaque bord latéral, lesquelles au moins deux parties de coin opposées (20 et 21) se chevauchent lorsque la couche est portée, au moins un dispositif d'attache extérieur (28) fixé de manière permanente dans le voisinage immédiat d'un dit bord latéral de ladite couche au niveau d'une partie de coin (20) desdites au moins

9

deux parties de coin opposées (20 et 21) pour attacher ladite une desdites deux parties de coin (20 et 21) dans une disposition de chevauchement avec l'autre partie de coin (21) desdites deux parties de coin (20 et 21) par une fixation amovible dudit au moins un dispositif d'attache extérieur (28) sur ladite une partie de coin (20) desdites deux parties de coin (20 et 21) à une partie de panneau avant (22) dudit voile arrière adjacent auxdites autres parties de coin (21), et au moins un dispositif d'attache intérieur (26) fixé de manière permanente audit voile arrière (14) de façon adjacente audit un côté latéral de ladite couche (10) seulement dans ladite autre partie de coin (21) pour assujettir le voile supérieur non tissé (12) de ladite une partie de coin (20) audit voile arrière (14) de ladite autre partie de coin (21), ledit au moins un dispositif d'attache intérieur (26) comprenant un fond (30, 30') ayant deux faces, une face attachée audit voile arrière (14) et une seconde face caractériseé en ce que la seconde face a une région à tiges (36, 36') comprenant un réseau de tiges thermoplastiques dressées (36, 36') réparties sur au moins ladite seconde face, lesdites tiges se terminant par des moyens de pointe (35, 35') afin de pénétrer dans ledit voile supérieur (12) non tissé, lesdites tiges (36, 36'), y compris lesdits moyens de pointe (35,35'), n'ayant pas de saillie sur elles pour accrocher des fibres, ledit au moins un dispositif d'attache intérieur situé sur le voile arrière de manière à permettre la pénétration dudit voile supérieur non tissé (12) ou d'un substrat perforé (24) sur ledit voile non tissé au niveau d'une dite partie de coin (20) par lesdites tiges (36) de ladite région à tiges lorsque ledit au moins un dispositif d'attache extérieur (28) est fixé à ladite partie de panneau avant (22).

2. Couche culotte selon la revendication 1, caractérisée en ce que lesdites tiges (36, 36') s'amenuisent vers l'intérieur en s'éloignant de ladite seconde face de fond (30, 30').

3. Couche culotte selon la revendication 2, caractérisée en ce que lesdites tiges (36) sont sensiblement coniques ou pyramidales.

4. Couche culotte selon la revendication 1, caractérisée en ce que lesdites tiges (36) ne s'amenuisent sensiblement pas (34') à partir de ladite seconde face de fond.

5. Couche culotte selon la revendication 1, caractérisée en ce que lesdites tiges (36, 36') font 130 à 510 $\mu$m depuis lesdits moyens de pointe (35, 35') jusqu'au plan de ladite seconde face de fond (30, 30').

6. Couche culotte selon la revendication 1, dans laquelle ledit dispositif d'attache intérieur (26) comprend une attache mécanique et adhésive (40), ladite seconde face comprend une région contenant de l'adhésif (41) et la région à tiges (42) est adjacente à ladite région contenant de l'adhésif (41).

Fig. 1

Fig. 2

11

35

34

36

38          26          30

*Fig. 3*

40

41          42

*Fig. 5*

35'

34'          36'

30'

26'          38'

*Fig. 4*